# EUROPEAN PATENT SPECIFICATION

(11) **EP 1 516 579 B1**
(45) Date of publication and mention of the grant of the patent: **24.09.2008**
(21) Application number: 04255553.2
(22) Date of filing: 14.09.2004
(51) Int. Cl.: A61B 5/00

(54) **Apparatus and method for measuring blood components**
Vorrichtung und Verfahren zur Bestimmung von Blutkomponenten
Appareil et procedé pour mesurer des composants sanguins

(30) Priority: 16.09.2003 KR 2003064208
(43) Date of publication of application: 23.03.2005
(73) Proprietor: SAMSUNG ELECTRONICS CO., LTD., Suwon-si, Gyeonggi-do (KR)
(72) Inventor: Jeon, Kye-jin, Suwon-si Gyeonggi-do (KR); Choe, Eun-young, Suwon-si, Gyeonggi-do (KR); Hwang, In-duk, Suwon-si Gyeonggi-do (KR); Kim, Hye-jeong, Samsung Advanced Inst. of Tech., Yongin-si Gyeonggi-do (KR)
(74) Representative: Greene, Simon Kenneth

(56) References cited:
- EP-A- 1 459 679
- WO-A-03/039326
- US-A- 5 551 422
- US-A- 6 119 026

## Description

The present invention relates to an apparatus and method for measuring bio-tissue components, and more particularly, to an apparatus and method for measuring blood components such as blood sugar.

A human body consists of 73% water and 27% other components. 1/3 of the water is extracellular, while 2/3 is intracellular. Among the extracellular water, 3/4 is interstitial fluid, while 1/4 is intravascular fluid. The blood sugar refers to the concentration of glucose in blood. The concentration of glucose contained in the blood flowing along a capillary vessel is similar to that of the interstitial fluid.

The human body tissue consists of flexible cells between which the interstitial fluid exists. Therefore, when external pressure is applied to the tissue, the tissue is depressed and the interstitial fluid travels.

When a spectrum of a blood component is measured, the measured result may vary in accordance with a variety of conditions such as a surface state of a measured tissue, pressure applied to the tissue, and the like. Therefore, in order to predict the concentration of a specific component in the tissue, there is a need for an active control of the measured tissue. In addition, it is methodologically important how to control the tissue measured.

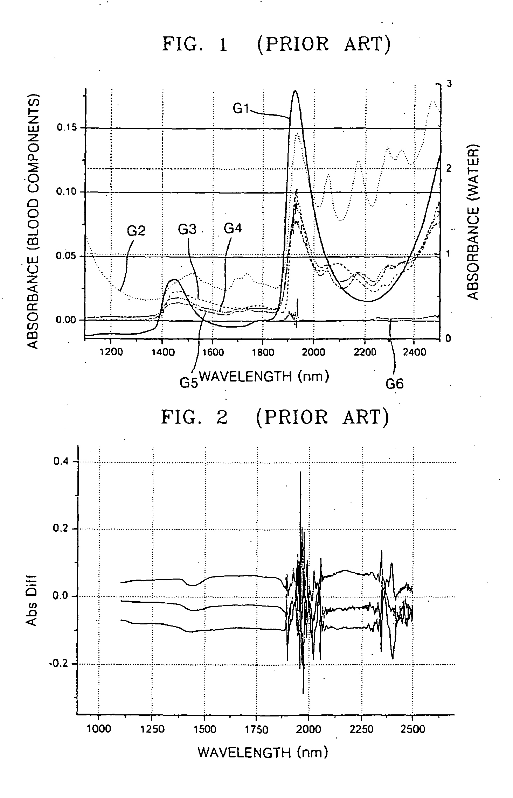

FIGS. 1 and 2 show why such an active control and control method are important.

FIG. 1 shows absorbance spectrums of blood components such as glucose, hemoglobin, albumin, triacetine, and gamma (y )-globulin and an absorbance spectrum of water. The absorbance spectrums G2, G3, G4, G5, and G6 of hemoglobin, glucose, albumin, triacetine, and gamma (y )-globulin are obtained by extracting the absorbance spectrum of the water from an absorbance spectrum (not shown) of aqueous solution of each component having a path length of 0.5 mm with respect to a near infrared ray.

In FIG. 1, the left-longitudinal axis represents each absorbance of major blood components and the right-longitudinal axis represents an absorbance of the water.

As shown in FIG. 1, the absorbance of the water at each of the wave bands 1600 nm and 2200 nm is greater than those of other blood components by more than 20 times. It can be further noted that the near infrared ray absorption band of the glucose and the near infrared ray absorption bands of other blood components overlap one another.

As optical technologies and statistical analysis technologies are advancing, active researches are being conducted for measuring the blood sugar using light in the near infrared ray range without using the blood. However, there has been no satisfactory result due to a variety of causes such as a light scattering, a relatively high absorbance of the water, an interference caused by the overlap of the near infrared ray absorption bands of the glucose and other blood components, and diversity of the tissue to be examined.

Accordingly, when the blood components are measured using the light in the near infrared ray range, it is required to properly control the tissue to be examined in a direction where a signal-to-noise ratio is increased by enlarging variation of the body fluid while correcting the influence of the tissue that may vary each time the measurement is performed, considering the results shown in FIG. 1.

The inventor of the present invention measured an absorbance of an aqueous solution having a thickness of 0.5 mm and containing 500 mg of glucose using a conventional apparatus and method. Only the absorbance of the glucose can be obtained by extracting the absorbance of the water from the whole absorbance of the aqueous solution.

The measured results are shown in Table 1. The measured results shown in Table 1 correspond to the absorbance of the glucose contained in a soft tissue having a thickness of 2 mm.

**[Table 1]**

| Wavelength (nm) | 1689 (peak) | 2094 (peak) | 2238 (valley) | 2274 (peak) | 2360 (peak) |
|---|---|---|---|---|---|
| Absorbance | 0.0006 | 0.0046 | 0.00209 | 0.00246 | 0.00507 |

The inventor further measured the absorbance variation of a 1.7 mm thickness web tissue between the thumb and the index finger using the conventional apparatus and method. FIG. 2 shows the measured results.

Through the measured results shown in FIG. 2, the absorbance variation of the web tissue having a thickness greater than 1.7 mm can be assumed.

Referring to FIG. 2, it can be noted that, when light having a wavelength of 1650 nm is used, the absorbance variation is about ±0.03 Abs. It can be further noted that, when light having a wavelength of 2200 nm is used, the absorbance variation is about ±0.05 Abs.

When comparing results shown in Table 1 with the results of FIG. 2, it can be noted that, when the light having the wavelength of 1650 nm is used, the repeated measuring error (±0.03 Abs) is more than 100 times greater than the absorbance (0.0006 Abs) of the glucose. It can be further noted that, when the light having the wavelength of 2200 nm is used, the repeated measuring error (0.05 Abs) is 40 times greater than the absorbance (0.00209 Abs).

As described above, when the blood components such as the blood sugar are measured without properly controlling the tissue measured, the absorbance variation according to the repeated measurements becomes much greater than the actual absorbance of the tissue to be examined. When the absorbance variation is increased, the reproducibility is deteriorated. That is, the absorbance of a tissue measured may vary whenever being measured. As a result, the absorbance measuring results are not reliable and the blood component data obtained through the absorbance analysis are indefensible.

A prior art non-invasive apparatus for measuring blood is described in WO 03/039326.

According to an aspect of the present invention, there is provided a blood component measuring apparatus according to claim 1.

The present invention provides a blood component measuring apparatus that can measure blood components such as blood sugar without using blood and can minimize absorbance variation (i.e., the measurement error) that may be caused by variation of an interfacing between the measuring apparatus and the tissue to be examined.

The first and second fixing members may be provided on the same base plate so that the first and second fixing members are pivotted on their axes fixed on the same base plate.

The first fixing member may comprise a first support pivotable on a fixed portion; a movable member mounted on the first support and providing a space where the part connected to the tissue of the examinee's body part is located, a portion of the movable member being movable to vary the size of the space in response to a size of the part connected to the tissue of the examinee's body part; and an adjustor for adjusting movement of the movable member.

The first fixing member may further comprise a catching means for pulling the part connected to the tissue of the examinee's body part downward or outward.

The adjustor may comprise a first adjustor for increasing the space in a longitudinal direction of the first portion of the examinee's body part and a second adjustor for increasing the space in a perpendicular direction to the longitudinal direction of the part connected to the tissue of the examinee's body part.

The blood component measuring apparatus may further comprise a stopper for stopping a pivotal motion of the first support.

The second fixing member may comprise a second support pivotable on a fixed portion; a supporting member mounted on the second support to support the other part connected to the tissue of the examinee's body part, the supporting member being movable to meet a size of the other part of the examinee's body part; a catcher for pulling a portion of the tissue to be examined; a movable member for moving the catcher; and an adjustor for adjusting movement of the supporting member.

The catcher may comprise an upper member and a lower member contacting.

The blood component measuring apparatus may further comprise a stopper for stopping rotation of the second support.

The light source portion may comprise: a light source for irradiating the light and a photo-guider for guiding the light irradiated from the light source portion to the tissue to be examined.

The photo-guider may be provided with a heater for adjusting a temperature of the tissue to be examined. Preferably, the heater is formed on a surface of the photo-guider or formed enclosing the photo-guider.

The photo-detector may comprise: a photo-guide pillar contacting the tissue to be examined to guide the light passing through the tissue to be examined; and a protecting tube enclosing the photo-guide pillar and make an interfacing area between the photo-guide pillar and the tissue to be examined gentle. The protecting tube comprises: a portion in parallel with the photo-guide pillar and a portion bent from the parallel portion toward a lower end of the photo-guide pillar. The bent portion is inclined at an angle of 30-60°with respect to a horizontal plane which is perpendicular to the photo-guide pillar is disposed.

According to another aspect of the present invention, there is provided a blood component measuring method using the above-described apparatus, the method comprising: mounting the examinee's body part on the fixing apparatus such that the light source portion contact a first surface of the tissue to be examined of the examinee's body part; uniformly maintaining a temperature of the tissue to be examined of the examinee's body part at a predetermined temperature; applying a tension to the tissue to be examined; and obtaining desired data from the tissue to be examined.

Another object of the present invention is to provide a blood component measuring method using the apparatus.

The step of applying a tension may be performed before the step of uniformly maintaining a temperature.

The temperature of the tissue to be examined may be maintained in a range of 36-40°C.

The tension may be applied by spreading opposing portions of the examinee's body part. The tension may be further applied by pulling the tissue to be examined in a state where the opposing portions of the examinee's body part are spread. In addition, the tension may be further applied by pulling or pressing a first region adjacent to a target region of the tissue to be examined where the measurement is realized. The tension may be further applied by pulling or pressing a second region adjacent to a target region of the tissue to be examined where the measurement is realized.

The blood component measuring method may further comprise fixing the examinee's body part itself.

The obtaining desired data further comprises: contacting the photo-detector with a second surface of the tissue to be examined facing the light source portion; irradiating the light to one side of the tissue to be examined and detecting the light passing through the tissue to be examined; amplifying the detected light; and analysing the amplified light to output data on the blood component in the tissue to be examined.

A contacting portion between a periphery of the photo-detector and the tissue to be examined is inclined at an angle of a range 30-60°.

The first region is pressed by pressure equal to or less than 0.5 N/mm².

According to the above-described invention, the absorbance variation (i.e., the absorbance measuring error) during the absorbance measuring process for measuring the blood components can be reduced. As a result, the reproducibility with respect to the absorbance measuring condition can be enhanced, thereby improving the reliability of blood components data obtained through the absorbance analysis.

The above and other features and advantages of the present invention will become more apparent by describing in detail exemplary embodiments thereof with reference to the attached drawings in which:
FIG. 1 is a graph illustrating absorption spectrums of major blood components;
FIG. 2 is a graph illustrating absorbance variation of a web tissue having a thickness of 1.7 mm;
FIGS. 3a and 3b are graphs illustrating absorbance variation in accordance with control parameters relating to an absorbance measurement;
FIG. 4 is a block diagram of a blood component measuring apparatus according to a preferred embodiment of the present invention;
FIG. 5 is a perspective view of an examinee's body part fixing device depicted in FIG. 4;
FIG. 6 is a view of an examinee's body part that will be fixed on an examinee's body fixing device depicted in FIG. 5;
FIG. 7 is a sectional view of a photo-detector depicted in FIG. 4;
FIG. 8 is a block diagram for explaining a blood component measuring method using an apparatus depicted in FIG. 4;
FIG. 9 is a block diagram illustrating a second operation depicted in FIG. 8; and
FIG. 10 is a block diagram illustrating a third operation depicted in FIG. 8.

The present invention will now be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete. In the drawings, the thicknesses of layers and regions are exaggerated for clarity.

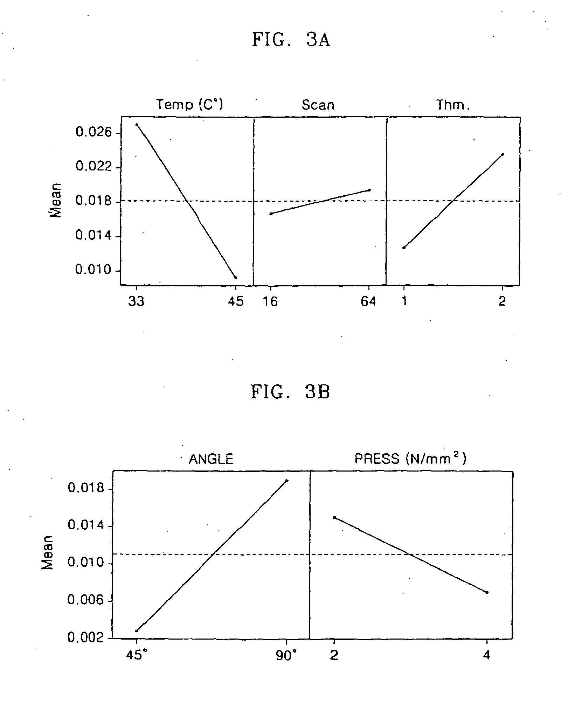

The inventor tested how factors affecting the measurement of the blood components affect the absorbance measurement. FIGS. 3a and 3b show the test results.

In FIG. 3a, a left portion shows an absorbance variation of a tissue to be examined in response to temperature variation, a middle portion shows an absorbance variation in accordance with the number of scans, and a right portion shows an absorbance variation according to thickness variation of the tissue to be examined.

In FIG. 3b, a left portion shows an absorbance variation in accordance with inclined angle variation of a periphery of a photo-detector for detecting light passing through the tissue to be examined, and a right portion shows an absorbance variation in response to variation of pressure applied to the tissue to be examined.

Referring to FIGS. 3a and 3b, it can be noted that the temperature of the tissue to be examined is the factor most affecting the absorbance variation, while the number of scans does not affect much the absorbance variation.

That is, the test results show that the temperature of the tissue to be examined becomes the most important factor for measuring the blood components. Accordingly, it is preferable to maintain the temperature of the tissue to be examined within a predetermined range. In order to enhance the reproducibility of the tissue to be examined, it is further preferable that the temperature of the tissue to be examined be maintained as high as possible.

However, when it is taken into account that the tissue to be examined is a portion of a human body, there is a limitation in increasing the temperature of the tissue to be examined. Therefore, it is preferable to maintain the temperature of the tissue to be examined to be less than, for example, 40 °C, more preferably, to be in a range of 36-40 °C.

Secondly, the absorbance is affected by an angle between peripheries of the photo-detector and the tissue to be examined. The absorbance variation caused by the angle can be attenuated by preventing a shape of the tissue to be examined from being steeply varied. This will be described more in detail later.

When light transmitting the tissue to be examined is measured, the reproducibility of the measurement can be improved by thinning the tissue to be examined. However, it is difficult to thin the tissue to be examined when it is taken into account that the tissue to be examined is a portion of the human body.

Generally, an examinee feels a pain when a pressure applied to the tissue to be examined becomes greater than 0.5 N/mm². Therefore, it is preferable that the pressure applied to the tissue to be examined be less than 0.5 N/mm².

Meanwhile, by making a thickness of the tissue to be examined uniform by uniformly spreading the tissue to be examined, it is also possible to improve the absorbance reproducibility. That is, to spread the tissue to be examined, it is preferable that relatively high pressure be applied to the tissue to be examined. However, it is preferable that the pressure be less than a predetermined value so that the examinee does not feel the pain.

Therefore, the inventive apparatus has been made considering the above-described factors.

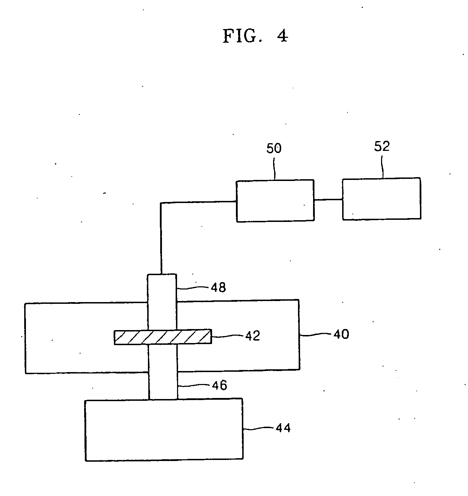

FIG. 4 shows a blood component measuring apparatus according to a preferred embodiment of the present invention.

To minimize the absorbance variation, the present invention improves an interfacing between a part of human body and an apparatus. The interfacing will be hereinafter called "IHA."

The IHA can be improved by (a) maintaining a temperature of the tissue to be examined as high as possible during the measurement, (b) applying a pressure to maintain a thickness of the tissue to be examined as thin as possible, (c) forming the angle as small as possible, and (d) fixing a light irradiation location during the measurement.

Referring to FIG. 4, the measuring apparatus includes a fixing device for fixing an examinee's body part. A photo-guider 46 and a photo-detector 48 are respectively located on top and bottom of a tissue to be examined 42 of the examinee's body part. Preferably, the photo guider 46 and the photo-detector 48 are disposed facing each other. The photo guider 46 functions to guide light irradiated from a light source 44 to a target point of the tissue to be examined 42. The light source 44 and the photo-guider 46 constitute a light source portion. The photo-guider 46 is provided at a surface with a heater 46a for uniformly maintaining a temperature of the tissue to be examined in a range of, for example, 36-40 °C. The heater 46a may be provided in the form of enclosing the photo-guider 46. The photo-detector 48 detects light passing through the tissue to be examined 42. The photo-detector 48 is connected to an amplifier 50 connected to an analyzer 52. The amplifier 50 functions to amplify signal outputted from the photo-detector 48 and the analyzer 52 analyses the amplified signal outputted from the amplifier 50 to output data on the blood component such as the blood sugar contained in the tissue to be examined 42.

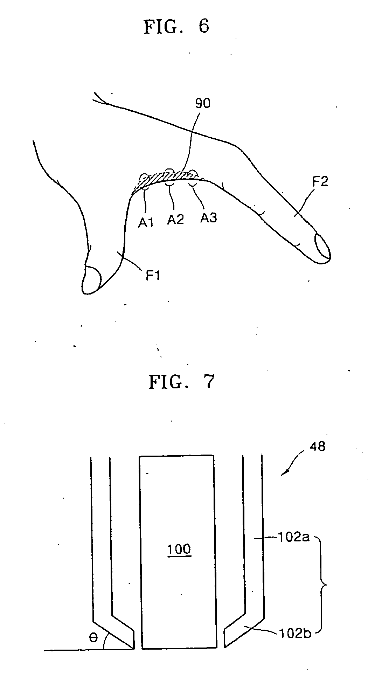

Meanwhile, as shown in FIG. 6, a tissue 90 between a thumb F1 and an index finger F2 is preferable as the tissue to be examined 42. However, other portions such as an earlobe, a lip, an eyelid, a spread skin, and the like that are relatively thin as compared with other portions can also be used as the tissue to be examined 42.

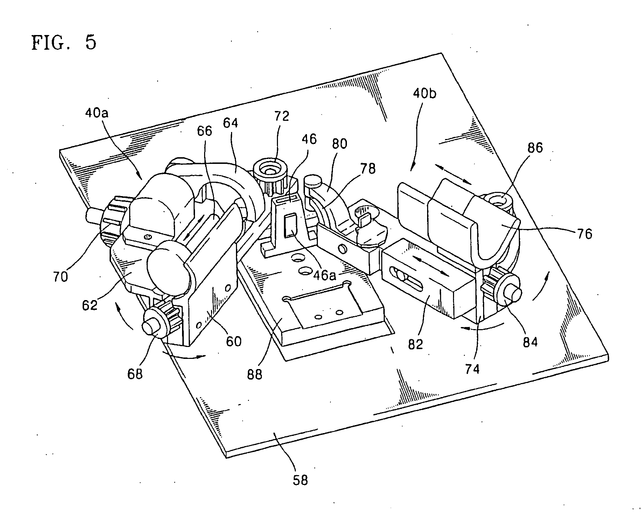

The fixing device 40 for fixing the examinee's body can be formed in a variety of structures in accordance with the shape of the tissue to be examined 42 of the examinee's body part. When the tissue to be examined 42 is the tissue 90 between the thumb F1 and the index finger F2 (see FIG. 6), the fixing device 42 is formed as shown in FIG. 5.

In more detail, the fixing device 40 shown in FIG. 5 is for a left-handed person. The fixing device 40 comprises a thumb fixing member 40a and an index finger fixing member 40b that are provided on a base plate 58. The thumb and index finger fixing members 40a and 40b are widened from each other at a predetermined angle. One section of the thumb fixing member 40a is fixed on the base plate 58 by a bolt 72 while the other section of the thumb fixing member 40b is pivotable at a predetermined angle base plated on the fixed section. One section of the index finger fixing member 40b is fixed near the thumb fixing member 40a on the base plate 40a by a bolt (not shown), and the other section of the index finger fixing member 40b is pivotable by a predetermined angle with respect to a base plated on the fixed section. The photo-guider 46 is disposed between the thumb and index finger fixing members 40a and 40b, and the heater 46a is formed on the surface of the photo-guider 46.

When the thumb F1 and the index finger F2 are respectively fixed on the thumb and index finger fixing members 40a and 40b, the tissue 90 between the thumb F1 and the index finger F2 is disposed on a top of the photo-guider 46 to cover a light emission hole through which the light is emitted. At this point, it is preferable that a first region A1 of the tissue 90 is located on the center of the light emission hole. The first region A1 is a region through which the light emitted from the light emission hole passes to allow the absorbance for measuring the blood component to be measured.

By spreading the thumb F1 from the index finger F2 at a predetermined angle using the thumb and index finger fixing members 40a and 40b, it is possible to apply a predetermined tension to web tissue 90. At this point, it is preferable that the predetermined tension is set as high as possible within a range where the examinee does not feel the pain so that the absorbance can be more accurately measured by forming the thickness of the tissue 90 as thin as possible. The thickness of the tissue can be further reduced by applying a predetermined pressure to a predetermined region of the tissue 90 such as second and third regions A2 and A3. The pressure can be applied to one of or both the second and third regions A2 and A3. The pressure can be applied to depress the second and third regions A2 and A3 downward or to pull the same frontward.

Referring again to FIG. 5, the thumb fixing member 40a comprises a first support 60 having a first section fixed on the base plate 58 and a second section pivotable base plated on the fixed portion and a movable member 62 supported by the first support 60 to adjust a space of a thumb receiving region 66. The movable member 62 is designed to move in a direction where the thumb receiving region 66 can be lengthened or shortened in accordance with a length of the thumb and to move in a direction where the thumb receiving region 66 can be widened or narrowed in accordance with a thickness of the thumb. A first adjustor 68 is provided on, for example, one end of the first support 60. While rotating clockwise or counterclockwise, the first adjustor 68 moves the movable member 62 so that the thumb receiving region 66 can meet the length of the thumb. A second adjustor 70 is provided on, for example, one side of the movable member 62. While rotating clockwise or counterclockwise, the second adjustor 70 adjusts the thumb receiving region 66 such that the thumb receiving region 66 can be widened or narrowed in response to the thickness of the thumb. The reference numeral 64 indicates a first pressing member for applying pressure to the second region A1. The first pressing member 64 is pivotally fixed in a vertical direction. That is, in order to allow the thumb to be placed in the thumb receiving region 66, the first pressing member 64 is first pivoted upward so that the thumb can received in the thumb receiving region 66, after which the first pressing member 64 is pivoted downward to apply predetermined force to the second region A2 of the tissue 90 shown in FIG. 6).

A stopper (not shown) for stopping the thumb fixing member 40a from pivoting at a predetermined angle may be placed under the second adjustor 70.

The index finger fixing member 40b comprises an index finger support 76 for receiving the index finger, tissue catchers 78 and 80 for fixing the third region A3 of the tissue 90 shown in FIG. 6, a movable member 82 for moving the tissue catchers 78 and 80, and a second support 74 for supporting these elements. The ) second support 74 is pivotally fixed on the base plate 58. The index finger support 76 is designed to be movable in response to a length of the index finger. To move the index finger support 76, a third adjustor 84 is provided on one end of the second support 74. While rotating clockwise or counterclockwise, the third adjustor 84 moves the index finger support 76 toward or away from a fixing portion of the second support 74. First corresponding ends of the tissue catchers 78 and 80 contact each other when the tissue 90 is not fitted between them. When the thumb F1 and the index finger F2 are respectively placed in the thumb and index finger fixing members 40a and 40b, second corresponding ends of the tissue catchers 78 and 80, which are opposite to the first corresponding ends, are pushed to thereby define a predetermined gap between the first corresponding ends of the tissue catchers 78 and 80. In this state, the third region A3 of the tissue 90 is fitted in the gap defined between the first corresponding ends of the tissue catchers 78 and 80, after which, when the pushing force is released, the third region A3 of the tissue 90 is fixed by a pressing force of the tissue catchers 78 and 80. The tissue catchers 78 and 80 are disposed on an identical line where the movable member 82 is disposed so that the tissue catchers 78 and 80 can move in a direction where the movable member 82 moves. For example, there is a need for providing predetermined tension to the tissue 90. That is, after the third region A3 of the tissue 90 is fixed between the tissue catchers 78 and 80, the tissue catchers 78 and 80 are moved away from the tissue 90 by moving the movable member 82 outward to apply the tension to the tissue 90. To prevent the movable member 82 from displacing after the movable member 82 moves by a predetermined distance, a stopper (not shown) and a stopper release member (not shown) can be further provided. After the measurement for the tissue 90 is finished or in order to return the tissue catchers 78 and 80 to their initial locations, the stopper is released by the stopper release member. In addition, after the second support 74 on which the tissue catchers 78 and 80, the movable member 82 and the index finger support 76 are mounted is pivoted at a predetermined angle, the second support 74 is maintained at its pivoted angle by a stopper 86. The stopper 86 allows the second support 74 to be screw-coupled on the base plate 58,

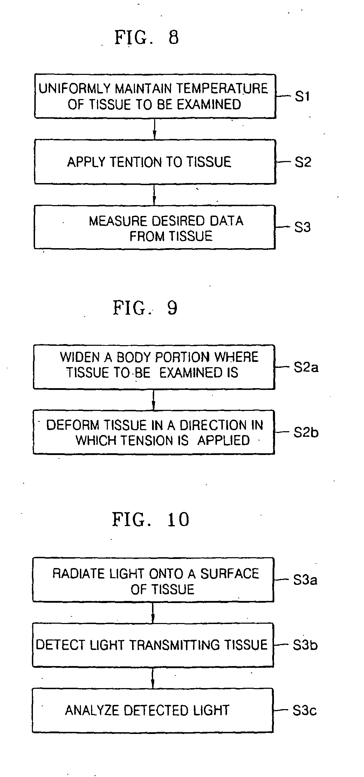

The reference numeral 88 in FIG. 8 indicates a base plate for attaching the photo-guider 46 to the light source (44 in FIG. 4) disposed under the photo guider 46. The thumb and index finger fixing members 40a and 40b and the photo-guider 46 can be mounted on a single base plate attached on the light source 44. Alternatively, in a state where the photo-guider 46 is directly attached on the light source 44, a single base plate provided with a hole through which the photo-guider 46 passes and on which the thumb and index finger fixing members 40a and 40b are mounted can be attached on the light source 44.

The photo-detector 48 contacting the top of the first region A1 of the tissue 90, as shown in FIG. 7, comprises a photo-guide pillar 100 and a protecting tube 102 disposed at a predetermined interval around the photo-guide pillar 100. The photo-guide pillar 100 functions to guide the light emitted from the photo-guider 46 and transmit the first region A1 of the tissue 90 to the amplifier 50. The photo-guide pillar 100 is formed in a cylindrical shape of circular or oval sections, or a prism, preferably, in a rectangular prism. The protecting tube 102 protects the photo-guide pillar 100 and makes an interfacing area between the photo-detector 48 and the first region A1 gentle. As a result, since a portion of the first region A1, which is defined on an inner side of the protecting tube 102, becomes flat, the photo-guide pillar 100 can contact the flat first region A1. The protecting tube 102 comprises a portion 102a in parallel with the photo-guide pillar 100 and a portion 102b bent from the parallel portion 102a toward a lower end of the photo-guide pillar 100. The bent portion 102b extends to almost reach the photo-guide pillar 100. The bent portion 102b is inclined at a predetermined angle θ with respect to a horizontal plane on which a bottom of the photo-guide pillar 100 is disposed.

When the tissue to be examined is the web-tissue 90 shown in FIG. 6, it is preferable that the predetermined inclined angle θ be equal to or less than 60°, more preferably, be in a range of 30-60 °.

When the tissue to be examined is not the web-tissue 90 but other tissue, the inclined angle may be varied.

In addition, a hand fixing member may be further provided. That is, in a state where the thumb F1 and the index finger F2 are respectively fixed on the thumb and index finger fixing members 40a and 40b, the hand, for example, a wrist can be fixed by the hand fixing member.

A blood component measuring method using the above-described apparatus will be described hereinafter.

Referring first to FIG. 8, the blood component measuring method of the present invention is comprised of first to third operations S1, S2, and S3.

The first operation S1 is for adjusting a temperature of the tissue to be examined of the examinee (i.e., the first region A1 of the tissue 90 shown in FIG. 6) to a predetermined temperature. The temperature of the tissue to be examined is adjusted by the heater 46a provided on the photo-guider 46. At this point, although it is preferable to maintain a temperature of the tissue to be examined as high as possible, when it is taken into account that the tissue to be examined is a portion of the human body, it is preferable that the temperature of the tissue to be examined is uniformly maintained in a range of, for example, 36-40 °C.

Before the first operation S1 is performed, the thumb F1 and the index finger F1 are respectively fixed on the thumb and index finger fixing members 40a and 40b and the tissue 90 between the thumb F1 and the index finger F1 is located on the top of the photo-guider 46, in the course of which, by operating the first to third adjustors 68, 70 and 84, the thumb and the index finger are comfortably fixed on the thumb and index finger fixing members 40a and 40b. In addition, using the hand fixing member, the hand (the wrist) can be also fixed.

The second operation S2 is for adjusting tension of the tissue to be examined to a predetermined level.

The order of performing the first and second operations S1 and S2 can be changed. That is, after the tension of the tissue to be examined is adjusted, the temperature of the same can be adjusted.

FIG. 9 shows a detailed process of the second operation S2.

As shown in Fig. 9, the second operation S2 includes an operation S2a for spreading the tissue to be examined at a predetermined angle and an operation S2b for deforming the tissue to be examined in a direction where the tension occurs.

In the step S2a, it is preferable that the circumference of the tissue to be examined until the tissue to be examined is completely spread within a range where the examinee does not feel pain. The spreading of the tissue to be examined can be varied according to a kind of the tissue to be examined. For example, when the tissue to be examined is the tissue 90 between the thumb F1 and the index finger F2, the tissues 90 of the examinees may be different in a thickness from each other. Accordingly, it is preferable that the opening angle between the thumb F1 and the index finger F2 is varied according to each individual. However, it is preferable that the measuring condition of the tissue 90 be identically maintained.

In the operation S2b, tension is applied to a specific region of the tissue to be examined spread in the operation S2a.

In more detail, although the tissue to be examined is already, there is a need for further applying a tension to the tissue to be examined to adjust, for example, a thickness of the same.

There are two methods for applying the tension to the tissue to be examined.

In a first method, in a state where the thumb F1 is placed in the thumb receiving region 66 of the thumb fixing member 40a, a predetermined force is applied to a specific region, for example, to the second region A2 of the tissue 90 using the first pressing member 64. By the applied force, the second region A2 of the tissue 90 is depressed downward, thereby applying the tension to the tissue 90.

In a second method, after the thumb F1 is fixed on the thumb fixing member 40a, a pulling force is applied to the third region A3 using the tissue catchers 78 and 80. When the third region A3 is pulled, the tension is applied to the tissue 90.

Meanwhile, in order to apply the tension to the tissue 90, the first and second methods can be simultaneously used. That is, the pressing force can be applied to the second region A2 of the tissue 90 while the pulling force is applied to the third region A3 by the tissue catchers 78 and 80.

In the first method, instead of using the first pressing member 64 to apply force to the second region A2, a second tissue catcher (not shown) similar to the tissue catchers 78 and 80 can be used to full the second region A2.

The third operation S3 can be sub-divided into three operations as shown in Fig. 10.

That is, the third operation S3 includes: an operation S3a for irradiating light to the tissue to be examined, an operation S3b for detecting light transmitting the tissue to be examined, and an operation S3c for analysing the light detected in the operation S3b.

In more detail, in the operation S3a, the light is irradiated to the bottom of the tissue to be examined (i.e., a bottom of the first region A1 shown in Fig. 6), which contacts the top of the photo-guider 46. At this point, it is preferable that the light is of the near infrared ray. In the operation S3b, the photo-detector 48 closely contacts the top of the tissue to be examined, facing the photo-guider 46 (see FIG. 7). In this state, the photo-detector 48 detects the light containing blood component information in the tissue to be examined, the light being irradiated from the photo-guider 46 and passing through the tissue to be examined. A thickness of the tissue to be examined can be adjusted by adjusting pressure in the course of closely contacting the photo-detector 48 on the tissue to be examined. At this point, it is preferable that the thickness of the tissue to be examined is adjusted by applying a pressure of 0.5 N/mm². However, the pressure can be varied in accordance with the examinee. For example, when the examinee feels pain by the pressure of 0.5 N/mm² applied to the tissue to be examined, it is preferable that the pressure is less than 0.5 N/mm².

In the operation S3c for analysing the detected light, the light detected by the photo-detector 48 is amplified by the amplifier 50. The amplified light is analysed by the analyzer 52. The analyzer 52 outputs data on the blood components in the tissue to be examined.

A test for measuring the blood sugar of the tissue 90 depicted in FIG. 6 was performed to demonstrate the advantages of the present invention.

According to test results, it was noted that, when the near infrared ray having a wavelength of 1650 nm is used, the absorbance variation is about ±0.007 Abs. It was further noted that, when the near infrared ray having a wavelength of 2200 nm was used, the absorbance variation was about ±0.013 Abs.

Referring to Table 1 showing the comparison between the test results obtained by using the apparatus and method of the present invention and the test results obtained by using the conventional apparatus, it can be noted the absorbance variation measured by the apparatus and method of the present invention is much less than that measured by the conventional apparatus.

The apparatus of the present invention can be used in many applications for measuring a variety of components in the human body not only blood sugar by using light in all bands as well as near infrared rays.

Furthermore, the apparatus of the present invention is designed to use a reaction between the near infrared ray and the blood components. Therefore, the inventive apparatus can be employed to measure a bio signal such as a pulse and tissue hydration.

As described above, the apparatus of the present invention has an advantage of applying uniform tension to the tissue to be examined, while uniformly maintaining the thickness of the tissue to be examined. In addition, the contacting portion between a periphery of the photo-detector and the tissue to be examined can be formed having a gentle inclination, and the temperature of the tissue to be examined can be uniformly maintained within a predetermined range. Therefore, when the apparatus of the present invention is used to measure the blood components, the reproducibility with respect to the measuring condition can be enhanced, thereby minimizing the absorbance variation and improving the reliability of blood component data obtained through the analysis of the absorbance.

While the present invention has been particularly shown and described with reference to exemplary embodiments thereof, it will be understood by those of ordinary skill in the art that various changes in form and details may be made therein without departing from the scope of the present invention as defined by the following claims.

For example, although the index finger support 76 depicted in FIG. 5 is designed to simply support the index finger, it can be designed to completely enclose the index finger. Furthermore, the thumb and index finger fixing members 40a and 40b can be designed to be connected to each other in the form of a mitten. That is, the index finger fixing member 40b can be replaced with a fixing member in which all of fingers except for the thumb can be inserted.

## Claims

1. A blood component measuring apparatus comprising:
a fixing apparatus (40) for fixing an examinee's body part;
a light source portion (44, 46) for irradiating light to a tissue to be examined of the examinee's body part in a state where the light source portion is covered by the tissue to be examined;
a photo-detector (48) for detecting light passing through the tissue to be examined, the photo-detector facing the light source portion; and
an analyzer (52) for analyzing the light detected by the photo-detector,
wherein the fixing apparatus (40) comprises:
a first fixing member (40a) for fixing a part, which is connected to the tissue to be examined, of the examinee's body part; and
a second fixing member (40b) for fixing another part, which is connected to the tissue to be examined, of the examinee's body part;
**characterised in that** the first and second fixing members (40a, 40b) are provided on the same base plate (58) so that the first and second fixing members are pivotable on their axes fixed on the same base to move relative to a region where the tissue to be examined is to be located.

2. The blood component measuring apparatus of claim 1, wherein the first fixing member (40a) comprises:
a first support (60) pivotable on a fixed portion;
a movable member (62) mounted on the first support and providing a space where the part connected to the tissue is located, a portion of the movable member being movable to vary the size of the space in response to a size of the first part connected to the tissue of the examinee's body part; and
an adjustor (68, 70) for adjusting movement of the movable member.

3. The blood component measuring apparatus of claim 2, wherein the first fixing member (40a) further comprises a pressing means (64) for pressing the part connected to the tissue of the examinee's body part.

4. The blood component measuring apparatus of claim 2 or 3, wherein the first fixing member further comprises a catching means for pulling the part connected to the tissue of the examinee's body part downward or outward.

5. The blood component measuring apparatus of claim 2, 3 or 4, wherein the adjustor comprises a first adjustor (68) for increasing the space in a longitudinal direction of the part connected to the tissue of the examinee's body part and a second adjustor (70) for increasing the space in a perpendicular direction to the longitudinal direction of the part connected to the tissue of the examinee's body part.

6. The blood component measuring apparatus of claim 2, 3, 4 or 5, further comprising a stopper for stopping a pivotal motion of the first support.

7. The blood component measuring apparatus of any preceding claim, wherein the second fixing member (40b) comprises:
a second support (74) pivotable on a fixed portion;
a supporting member (76) mounted on the second support to support the other part connected to the tissue of the examinee's body part, the supporting member being movable to meet a size of the other part of the examinee's body part;
a catcher (78, 80) for pulling a portion of the tissue to be examined;
a movable member (82) for moving the catcher; and
an adjustor (84) for adjusting movement of the supporting member.

8. The blood component measuring apparatus of claim 7, wherein the catcher comprises an upper member (78) and a lower member (80).

9. The blood component measuring apparatus of claim 7 or 8, further comprising a stopper for stopping rotation of the second support.

10. The blood component measuring apparatus of any preceding claim, wherein the light source portion comprises a light source (44) for irradiating the light and a photo-guider (46) for guiding the light irradiated from the light source portion to the tissue to be examined.

11. The blood component measuring apparatus of claim 10, wherein the photo-guider (46) is provided with a heater (4a) for adjusting a temperature of the tissue to be examined during measurement.

12. The blood component measuring apparatus of claim 11, wherein the heater (4a) is formed on a surface of the photo-guider (46) or formed enclosing the photo-guider (46).

13. The blood component measuring apparatus of any preceding claim, wherein the photo-detector (47) comprises:
a photo-guide pillar (100) contacting the tissue to be examined to guide the light passing through the tissue to be examined; and
a protecting tube (102) enclosing the photo-guide pillar and make an interfacing area between the photo-guide pillar and the tissue to be examined gentle.

14. The blood component measuring apparatus of claim 13, wherein the protecting tube (102) comprises a portion (102a) in parallel with the photo-guide pillar and a portion (102b) bent toward a lower end of the photo-guide pillar.

15. The blood component measuring apparatus of claim 14, wherein the bent portion (102b) is inclined at an angle of 30-60° with respect to a horizontal plane which is perpendicular to the photo-guide pillar.

16. The blood component measuring apparatus of any preceding claim, wherein the examinee's body part is an examinee's hand, and the tissue to be examined is a web tissue between a thumb and an index finger of the hand.

17. The blood component measuring apparatus of claim 16, further comprising a fixing member for fixing the arm or the wrist.

18. A blood component measuring method using the apparatus claimed in claim 1, the method comprising:
mounting the examinee's body part on the fixing apparatus such that the light source portion contacts a first surface of the tissue to be examined of the examinee's body part;
uniformly maintaining a temperature of the tissue to be examined of the examinee's body part at a predetermined temperature;
applying a tension to the tissue to be examined; and
obtaining desired data from the tissue to be examined.

19. The blood component measuring method of claim 18, wherein the applying the tension is performed before the uniformly maintaining a temperature.

20. The blood component measuring method of claim 18 or 19, wherein the temperature of the tissue to be examined is maintained in a range of 36-40 °C.

21. The blood component measuring method of claim 18, 19 or 20, wherein the tension is applied by spreading opposing portions of the examinee's body part.

22. The blood component measuring method of claim 21, wherein the tissue to be examined is transformed by pulling in a state where the opposing portions of the examinee's body part is spread.

23. The blood component measuring method of claim 22, wherein the tissue to be examined is transformed by pulling or pressing a first region adjacent to a target region of the tissue to be examined where the measurement is realized.

24. The blood component measuring method of claim 22, wherein the tissue to be examined is transformed by pulling or pressing a second region adjacent to a target region of the tissue to be examined where the measurement is realized.

25. The blood component measuring method of any of claims 18 to 24, further comprising fixing the examinee's body part itself.

26. The blood component measuring method of any of claims 18 to 25, wherein the obtaining desired data further comprises:
contacting the photo-detector (48) with a second surface of the tissue to be examined facing the light source portion;
irradiating the light to one side of the tissue to be examined and detecting the light passing through the tissue to be examined;
amplifying the detected light; and
analysing the amplified light to output data on the blood component in the tissue to be examined.

27. The blood component measuring method of claim 26, wherein a contacting portion between a periphery of the photo-detector (48) and the tissue to be examined is inclined at an angle of a range 30-60°.

28. The blood component measuring method of claim 23, wherein the first region is pressed by a pressure equal to or less than 0.5 N/mm².

29. The blood component measuring method of claim 23, wherein the tissue to be examined is transformed by pulling or pressing a second region adjacent to the target region of the tissue to be examined where the measurement is realized.

## Patentansprüche

1. Vorrichtung zum Messen von Blutkomponenten, die Folgendes umfasst:
eine Fixierungsvorrichtung (40) zum Fixieren eines Körperteils eines Prüflings;
ein Lichtquellenteil (44, 46) zum Ausstrahlen von Licht auf ein zu untersuchendes Gewebe des Körperteils des Prüflings in einem Zustand, in dem der Lichtquellenteil von dem zu untersuchenden Gewebe bedeckt ist;
ein Lichtdetektor (48) zum Erfassen von Licht, das durch das zu untersuchende Gewebe verläuft, wobei der Lichtdetektor dem Lichtquellenteil zugewandt ist; und
ein Analysegerät (52) zum Analysieren des Lichts, das von dem Lichtdetektor erfasst wurde,
wobei die Fixierungsvorrichtung (40) Folgendes umfasst:
ein erstes Fixierungselement (40a) zum Fixieren eines Teils, das mit dem zu untersuchenden Gewebe des Körperteils des Prüflings verbunden ist; und
ein zweites Fixierungselement (40b) zum Fixieren eines anderen Teils, das mit dem zu untersuchenden Gewebe des Körperteils des Prüflings verbunden ist;
**dadurch gekennzeichnet, dass** das erste und das zweite Fixierungselement (40a, 40b) auf derselben Auflageplatte (58) vorgesehen sind, so dass das erste und das zweite Fixierungselement an ihren Achsen schwenkbar sind, die auf derselben Auflage fixiert sind, um sie relativ zu einem Bereich zu bewegen, in dem sich das zu untersuchende Gewebe befindet.

2. Vorrichtung zum Messen von Blutkomponenten nach Anspruch 1, wobei das erste Fixierungselement (40a) Folgendes umfasst:
eine erste Halterung (60), die an einem fixierten Teil schwenkbar ist;
ein bewegliches Element (62), das an der ersten Halterung montiert ist und einen Raum bereitstellt, in dem sich das zu untersuchende Gewebe befindet, wobei ein Teil des beweglichen Elements beweglich ist, um die Größe des Raums als Reaktion auf eine Größe des ersten Teils, das mit dem Gewebe des Körperteils des Prüflings verbunden ist, zu variieren; und
eine Einstellvorrichtung (68, 70) zum Einstellen der Bewegung des beweglichen Elements.

3. Vorrichtung zum Messen von Blutkomponenten nach Anspruch 2, wobei das erste Fixierungselement (40a) weiterhin ein Anpressmittel (64) zum Anpressen des Teils, das mit dem Gewebe des Körperteils des Prüflings verbunden ist, umfasst.

4. Vorrichtung zum Messen von Blutkomponenten nach Anspruch 2 oder 3, wobei das erste Fixierungselement weiterhin ein Greifmittel zum Ziehen des Teils, das mit dem Gewebe des Körperteils des Prüflings verbunden ist, nach unten oder nach außen umfasst.

5. Vorrichtung zum Messen von Blutkomponenten nach Anspruch 2, 3 oder 4, wobei die Einstellvorrichtung eine erste Einstellvorrichtung (68) zum Vergrößern des Raums in einer Längsrichtung des Teils, das mit dem Gewebe des Körperteils des Prüflings verbunden ist, und eine zweite Einstellvorrichtung (70) zum Vergrößern des Raums in einer senkrechten Richtung des Teils, das mit dem Gewebe des Körperteils des Prüflings verbunden ist, umfasst.

6. Vorrichtung zum Messen von Blutkomponenten nach Anspruch 2, 3, 4 oder 5, die weiterhin einen Stopper zum Stoppen einer Schwenkbewegung der ersten Halterung umfasst.

7. Vorrichtung zum Messen von Blutkomponenten nach einem der vorhergehenden Ansprüche, wobei das zweite Fixierungselement (40b) Folgendes umfasst:
eine zweite Halterung (74), die an einem fixierten Teil schwenkbar ist;
ein Tragelement (76), das an der zweiten Halterung montiert ist, um das andere Teil, das mit dem Gewebe des Körperteils des Prüflings verbunden ist, zu tragen, wobei das Tragelement beweglich ist, um einer Größe des anderen Teils des Körperteils des Prüflings zu entsprechen;
eine Greifvorrichtung (78, 80) zum Ziehen eines Teils des zu untersuchenden Gewebes;
ein bewegliches Element (82) zum Bewegen der Greifvorrichtung und
eine Einstellvorrichtung (84) zum Einstellen der Bewegung des Tragelements.

8. Vorrichtung zum Messen von Blutkomponenten nach Anspruch 7, wobei die Greifvorrichtung ein oberes Element (78) und ein unteres Element (80) umfasst.

9. Vorrichtung zum Messen von Blutkomponenten nach Anspruch 7 oder 8, die weiterhin einen Stopper zum Stoppen der Drehung der zweiten Halterung umfasst.

10. Vorrichtung zum Messen von Blutkomponenten nach einem der vorhergehenden Ansprüche, wobei der Lichtquellenteil eine Lichtquelle (44) zum Ausstrahlen des Lichts und einen Lichtleiter (46) zum Leiten des Lichts, das von dem Lichtquellenteil ausgestrahlt wird, zu dem zu untersuchenden Gewebe umfasst.

11. Vorrichtung zum Messen von Blutkomponenten nach Anspruch 10, wobei der Lichtleiter (46) mit einem Heizelement (4a) zum Einstellen einer Temperatur des zu untersuchenden Gewebes während einer Messung versehen ist.

12. Vorrichtung zum Messen von Blutkomponenten nach Anspruch 11, wobei das Heizelement (4a) auf einer Oberfläche des Lichtleiters (46) ausgebildet oder den Lichtleiter (45) umschließend ausgebildet ist.

13. Vorrichtung zum Messen von Blutkomponenten nach einem der vorhergehenden Ansprüche, wobei der Lichtdetektor (47) Folgendes umfasst:
eine Lichtleitsäule (100), die das zu untersuchende Gewebe berührt, um das Licht zu leiten, das durch das zu untersuchende Gewebe verläuft; und
eine Schutzröhre (102), die die Lichtleitsäule umschließt und eine Grenzfläche zwischen der Lichtleitsäule und dem zu untersuchenden Gewebe sanft gestaltet.

14. Vorrichtung zum Messen von Blutkomponenten nach Anspruch 13, wobei die Schutzröhre (102) einen Teil (102a), der parallel zu der Lichtleitsäule ist, und einen Teil (102b), der zu einem unteren Ende der Lichtleitsäule hin gebogen ist, umfasst.

15. Vorrichtung zum Messen von Blutkomponenten nach Anspruch 14, wobei der gebogene Teil (102b) in einem Winkel von 30 - 60° in Bezug auf eine horizontale Ebene, die zu der Lichtleitsäule senkrecht ist, geneigt ist.

16. Vorrichtung zum Messen von Blutkomponenten nach einem der vorhergehenden Ansprüche, wobei es sich bei dem Körperteil des Prüflings um eine Hand des Prüflings handelt und es sich bei dem zu untersuchenden Gewebe um ein Interdigitalgewebe zwischen einem Daumen und einem Zeigefinger der Hand handelt.

17. Vorrichtung zum Messen von Blutkomponenten nach Anspruch 16, die weiterhin ein Fixierungselement zum Fixieren des Arms oder des Handgelenks umfasst.

18. Verfahren zum Messen von Blutkomponenten unter Verwendung der Vorrichtung nach Anspruch 1, wobei das Verfahren Folgendes umfasst:
Lagern des Körperteils des Prüflinge auf der Fixierungsvorrichtung, so dass der Lichtquellenteil eine erste Oberfläche des zu untersuchenden Gewebes des Körperteils des Prüflings berührt;
konstantes Halten einer Temperatur des zu untersuchenden Gewebes des Körperteils des Prüflings auf einer vorher festgelegten Temperatur;
Anlegen einer Spannung auf das zu untersuchende Gewebe und Beziehen gewünschter Daten von dem zu untersuchenden Gewebe.

19. Verfahren zum Messen von Blutkomponenten nach Anspruch 18, wobei das Anlegen der Spannung vor dem konstanten Halten einer Temperatur durchgeführt wird.

20. Verfahren zum Messen von Blutkomponenten nach Anspruch 18 oder 19, wobei die Temperatur des zu untersuchenden Gewebes in einem Bereich von 36 - 40 °C gehalten wird.

21. Verfahren zum Messen von Blutkomponenten nach Anspruch 18, 19 oder 20, wobei die Spannung angelegt wird, indem gegenüberliegende Teile des Körperteils des Prüflings gespreizt werden.

22. Verfahren zum Messen von Blutkomponenten nach Anspruch 21, wobei das zu untersuchende Gewebe umgewandelt wird, indem es in einen Zustand gezogen wird, in dem die gegenüberliegenden Teile des Körperteils des Prüflings gespreizt werden.

23. Verfahren zum Messen von Blutkomponenten nach Anspruch 22, wobei das zu untersuchende Gewebe umgewandelt wird, indem ein erster Bereich neben einem Zielbereich des zu untersuchenden Gewebes, an dem die Messung vorgenommen wird, gezogen oder angepresst wird.

24. Verfahren zum Messen von Blutkomponenten nach Anspruch 22, wobei das zu untersuchende Gewebe umgewandelt wird, indem ein zweiter Bereich neben einem Zielbereich des zu untersuchenden Gewebes, an dem die Messung vorgenommen wird, gezogen oder angepresst wird.

25. Verfahren zum Messen von Blutkomponenten nach einem der Ansprüche 18 bis 24, das weiterhin das Fixieren des Körperteils des Prüflings selbst umfasst.

26. Verfahren zum Messen von Blutkomponenten nach einem der Ansprüche 18 bis 25, wobei das Beziehen gewünschter Daten weiterhin Folgendes umfasst:
Inberührungbringen des Lichtdetektors (48) mit einer zweiten Oberfläche des zu untersuchenden Gewebes, die dem Lichtquellenteil zugewandt ist;
Ausstrahlen des Lichts auf eine Seite des zu untersuchenden Gewebes und Erfassen des Lichts, das durch das zu untersuchende Gewebe verläuft;
Verstärken des erfassten Lichts und
Analysieren des verstärkten Lichts zu Ausgabedaten zu der Blutkomponente in dem zu untersuchenden Gewebe.

27. Verfahren zum Messen von Blutkomponenten nach Anspruch 26, wobei ein Berührungsteil zwischen einem Rand des Lichtdetektors (48) und dem zu untersuchenden Gewebe in einem Winkel in einem Bereich von 30 - 60° geneigt ist.

28. Verfahren zum Messen von Blutkomponenten nach Anspruch 23, wobei der erste Bereich mit einem Druck von gleich oder weniger als 0,5 N/mm² angepresst wird.

29. Verfahren zum Messen von Blutkomponenten nach Anspruch 23, wobei das zu untersuchende Gewebe umgewandelt wird, indem ein zweiter Bereich neben dem Zielbereich des zu untersuchenden Gewebes, an dem die Messung vorgenommen wird, gezogen oder angepresst wird.

## Revendications

1. Appareil de mesure de composants sanguins comprenant:
un appareil de fixation (40) pour fixer une partie du corps d'un sujet examiné :
une partie formant source de lumière (44, 46) pour irradier de lumière un tissu à examiner sur la partie du corps du sujet examiné, dans un état dans lequel la partie formant source de lumière est recouverte par le tissu à examiner ;
un photodétecteur (48) pour détecter la lumière qui passe à travers le tissu à examiner, le photodétecteur faisant face à la partie formant source de lumière ; et
un analyseur (52) pour analyser la lumière détectée par le photodétecteur,
dans lequel l'appareil de fixation (40) comprend :
un premier élément de fixation (40a) pour fixer une partie, qui est connectée au tissu à examiner, de la partie du corps du sujet examiné; et
un second élément de fixation (40b) pour fixer une autre partie, qui est connectée au tissu à examiner, de la partie du corps du sujet examiné;
**caractérisé en ce que** les premier et second éléments de fixation (40a, 40b) sont prévus sur la même embase (58) de sorte que les premier et second éléments de fixation peuvent pivoter sur leurs axes fixés à la même embase pour se déplacer relativement à une région dans laquelle est situé le tissu à examiner.

2. Appareil de mesure de composants sanguins selon la revendication 1, dans lequel le premier élément de fixation (40a) comprend :
un premier support (60), qui peut pivoter sur une partie fixe ;
un élément déplaçable (62) monté sur le premier support et qui constitue un espace où est placée la partie connectée au tissu, une partie de l'élément déplaçable pouvant être déplacée pour faire varier la dimension de l'espace en fonction d'une dimension de la première partie connectée au tissu de la partie du corps du sujet examiné ; et
un ajusteur (68, 70) pour ajuster le mouvement de l'élément déplaçable.

3. Appareil de mesure de composants sanguins selon la revendication 2, dans lequel le premier élément de fixation (40a) comprend de plus un moyen de pression (64) qui exerce une pression sur la partie connectée au tissu de la partie du corps du sujet examiné.

4. Appareil de mesure de composants sanguins selon la revendication 2 ou 3, dans lequel le premier élément de fixation comprend de plus un moyen de saisie pour tirer vers le bas ou vers l'extérieur la partie connectée au tissu de la partie du corps du sujet examiné.

5. Appareil de mesure de composants sanguins selon la revendication 2, 3 ou 4, dans lequel l'ajusteur comprend un premier ajusteur (68) pour augmenter l'espace dans une direction longitudinale de la partie connectée au tissu de la partie du corps du sujet examiné, et un second ajusteur (70), pour augmenter l'espace dans une direction perpendiculaire à la direction longitudinale de la partie connectée au tissu de la partie du corps du sujet examiné.

6. Appareil de mesure de composants sanguins selon la revendication 2, 3, 4 ou 5, qui comprend de plus une butée qui arrête un mouvement de pivot du premier support.

7. Appareil de mesure de composants sanguins selon l'une quelconque des revendications précédentes, dans lequel le second élément de fixation (40b) comprend :
un second support (74) pouvant pivoter sur une partie fixe ;
un élément de support (76) monté sur le second support pour supporter l'autre partie connectée au tissu de la partie du corps du sujet examiné, l'élément de support pouvant être déplacé pour correspondre à une dimension de l'autre partie de la partie du corps du sujet examiné ;
un moyen de saisie (78, 80) pour exercer une traction sur une partie du tissu à examiner ;
un élément déplaçable (82) pour déplacer le moyen de saisie ; et
un ajusteur (84) pour ajuster le mouvement de l'élément de support.

8. Appareil de mesure de composants sanguins selon la revendication 7, dans lequel le moyen de saisie comprend un élément supérieur (78) et un élément inférieur (80).

9. Appareil de mesure de composants sanguins selon la revendication 7 ou 8, qui comprend de plus une butée pour arrêter la rotation du second support.

10. Appareil de mesure de composants sanguins selon l'une quelconque des revendications précédentes, dans lequel la partie formant source de lumière comprend une source de lumière (44) pour irradier de lumière et un guide-lumière (46) qui guide la lumière rayonnée par la partie formant source de lumière jusqu'au tissu à examiner.

11. Appareil de mesure de composants sanguins selon la revendication 10, dans lequel le guide-lumière (46) est pourvu d'un dispositif de chauffage (4a) pour ajuster une température du tissu à examiner pendant les mesures.

12. Appareil de mesure de composants sanguins selon la revendication 11, dans lequel le dispositif de chauffage (4a) est formé sur une surface du guide-lumière (46) ou formé pour renfermer le guide-lumière (46).

13. Appareil de mesure de composants sanguins selon l'une quelconque des revendications précédentes, dans lequel le photodétecteur (47) comprend :
une colonne guide-lumière (100), en contact avec le tissu à examiner, pour guider la lumière qui passe à travers le tissu à examiner; et
un tube protecteur (102) qui renferme la colonne guide-lumière et crée une surface d'interfaçage douce entre la colonne guide-lumière et le tissu à examiner.

14. Appareil de mesure de composants sanguins selon la revendication 13, dans lequel le tube protecteur (102) comprend une partie (102a) parallèle à la colonne guide-lumière et une partie (102b) coudée vers une extrémité inférieure de la colonne guide-lumière.

15. Appareil de mesure de composants sanguins selon la revendication 14, dans lequel la partie coudée (102b) est inclinée selon un angle de 30 à 60° relativement à un plan horizontal qui est perpendiculaire à la colonne guide-lumière.

16. Appareil de mesure de composants sanguins selon l'une quelconque des revendications précédentes, dans lequel la partie du corps du sujet examiné est une main de ce sujet, et le tissu à examiner est un tissu de la commissure interdigitale entre le pouce et l'index de la main.

17. Appareil de mesure de composants sanguins selon la revendication 16, qui comprend de plus un élément de fixation pour fixer le bras ou le poignet.

18. Procédé de mesure de composants sanguins utilisant l'appareil revendiqué à la revendication 1, le procédé consistant à :
placer la partie du corps du sujet examiné sur l'appareil de fixation de sorte que la partie formant source de lumière entre en contact avec une première surface du tissu à examiner de la partie du corps du sujet examiné ;
maintenir uniforme, à une valeur prédéterminée, une température du tissu à examiner de la partie du corps du sujet examiné ;
exercer une traction sur le tissu à examiner ; et
obtenir les données souhaitées à partir du tissu à examiner.

19. Procédé de mesure de composants sanguins selon la revendication 18, selon lequel la traction est exercée avant que la température soit maintenue uniforme.

20. Procédé de mesure de composants sanguins selon la revendication 18 ou 19, selon lequel le tissu est maintenu à une température de 36 à 40°C.

21. Procédé de mesure de composants sanguins selon la revendication 18, 19 ou 20, selon lequel la traction est exercée en étirant des portions opposées de la partie du corps du sujet examiné.

22. Procédé de mesure de composants sanguins selon la revendication 21, selon lequel le tissu à examiner est transformé par traction jusqu'à un état dans lequel les portions opposées de la partie du corps du sujet examiné sont étirées.

23. Procédé de mesure de composants sanguins selon la revendication 22, selon lequel le tissu à examiner est transformé par une traction ou une pression exercée sur une première région adjacente à une région ciblée du tissu à examiner où la mesure est effectuée.

24. Procédé de mesure de composants sanguins selon la revendication 22, selon lequel le tissu à examiner est transformé par traction ou pression exercée sur une seconde région adjacente à une région ciblée du tissu à examiner où la mesure est effectuée.

25. Procédé de mesure de composants sanguins selon l'une quelconque des revendications 18 à 24, qui comprend de plus la fixation de la partie du corps proprement dite du sujet examiné.

26. Procédé de mesure de composants sanguins selon l'une quelconque des revendications 18 à 25, selon lequel l'acquisition des données souhaitées comprend de plus :
la mise en contact du photodétecteur (48) avec une seconde surface du tissu à examiner, qui fait face à la partie formant source de lumière ;
l'irradiation, par la lumière, d'un côté du tissu à examiner, et la détection de la lumière qui passe à travers le tissu à examiner ;
l'amplification de la lumière détectée ; et
l'analyse de la lumière amplifiée pour sortir les données sur le composant sanguin dans le tissu à examiner.

27. Procédé de mesure de composants sanguins selon la revendication 26, selon lequel une partie en contact entre une périphérie du photodétecteur (48) et le tissu à examiner est inclinée selon un angle compris entre 30 et 60°.

28. Procédé de mesure de composants sanguins selon la revendication 23, selon lequel la première région est soumise à une pression égale ou inférieure à 0,5 N/mm².

29. Procédé de mesure de composants sanguins selon la revendication 23, selon lequel le tissu à examiner est transformé par une traction ou une pression exercée sur une seconde région adjacente à la région ciblée du tissu à examiner où la mesure est effectuée.
